# EUROPEAN PATENT SPECIFICATION

(11) **EP 3 421 455 B1**
(45) Date of publication and mention of the grant of the patent: **27.03.2019**
(21) Application number: 17178755.9
(22) Date of filing: 29.06.2017
(51) Int. Cl.: C07D 211/56

(54) **IMPROVED PROCESS FOR THE PREPARATION OF CHIRAL 3-AMINO-PIPERIDINS, USEFUL INTERMEDIATES FOR THE PREPARATION OF TOFACITINIB**
VERBESSERTES VERFAHREN ZUR HERSTELLUNG VON CHIRALEN 3-AMINO-PIPERIDINEN, NÜTZLICHE ZWISCHENPRODUKTE ZUR HERSTELLUNG VON TOFACITINIB
PROCÉDÉ AMÉLIORÉ POUR LA PRÉPARATION DE3-AMINO-PIPERIDINS CHIRAUX, DES INTERMÉDIAIRES UTILES POUR LA PRÉPARATION DE TOFACITINIB

(43) Date of publication of application: 02.01.2019
(73) Proprietor: F.I.S.- Fabbrica Italiana Sintetici S.p.A., 36075 Montecchio Maggiore (VI) (IT)
(72) Inventor: Pastó Aguilà, Mireia, 08028 Barcelona (ES); Preciado Gallego, Sara, 08028 Barcelona (ES); Miserazzi, Emanuele, 40030 Grizzana Morandi Bologna (IT)
(74) Representative: Leganza, Alessandro

(56) References cited:
- WO-A2-2007/012953
- WO-A2-2010/123919
- US-A1- 2004 102 627

## Description

### Technical Field

The object of the present invention is an improved process for the synthesis of a key intermediate of the pharmaceutically active substance known as Tofacitinib or salt thereof.

### Background Art

3-amino-piperidine compounds represent valuable intermediates for the preparation of pharmaceutically active agents. For example, the Janus kinase 3 (JAK3) inhibitor named Tofacitinib, having the structural formula (I): 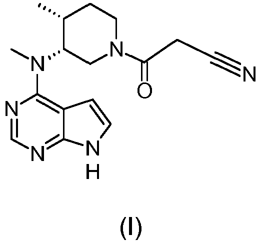
wherein both the asymmetric carbons have *R* configuration;
comprises a 4-methyl-3-(methylamino)piperidin-1-yl moiety having the structural formula

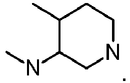

Janus kinase 3 (JAK3) inhibitors are a group of compounds that are classified to interfere with the Janus kinase signal transducer and activator of transcription (JAK-STAT) signalling pathway transmitting extracellular information into the cell nucleus and influencing DNA transcription.

Tofacitinib as one JAK3 inhibitor was found to be effective for many applications and can be used against e.g. rheumatoid arthritis, psoriasis, inflammatory bowel disease and other immunological diseases, as well as for prevention of organ transplant rejection.

Hu, et. al., Org. Lett. 2002, 4, pages from 4499 to 4502, describes a synthetic route for preparation of (3S)-amino-piperidine intermediates. In this synthetic route, predominantly products having trans-configuration of the substituents in 3 and 4 position of the piperidine ring are obtained. However, trans-configuration is not desired for intermediate compounds for preparing of Tofacitinib. Rather, cis- configuration is desired.

Brown, et. al, Org. Proc. Res. Dev. 2003, 7, pages from 115 to 120, and in particular at last part of second column of page 119 describes the preparation of 3-(methylamino)-4-methylpiperidine building block via reductive amination of 4-methylpiperidin-3-one using methylamine as reagent. The ketone was prepared by a combined hydroboration/oxidation process of methyl-tetrahydropyridine as describes in lorio, et. al., in Tetrahedron 1970, 26, page 5519 and Ripin, et. al., Tetrahedron Lett. 2000, 41, page 5817. The resulting compound was subjected to oxidation by an excess of SO₃ pyridine complex. The process involves application of hazardous reagents in the form of hydroborating agents such as NaBH₄ or BH₃ complexes and strong oxidants such as hydrogen peroxide, bleach or Oxone®.

Hao, et. al., Synthesis 2011, 8, pages 1208 to 1212, describes a synthetic route which starts from ethyl 1-benzyl-3-oxopiperidine-4-carboxylate hydrochloride. It is noteworthy to mention that the process is lengthy in terms of the amount of procedural steps required. Furthermore, the process requires hazardous and expensive reagents and starts from an advance intermediate. Asymmetric reduction of olefin in the presence of cobalt catalysts affords modest diastereomeric excess of 71%. Reductive amination to incorporate methyl group on amine part of molecule represents the key step, however, accomplishing this reductive amination is problematic. Besides, stereoselective transformation of ester group to methyl requires costly and hazardous reagents.

Furthermore, Cai. Et al.; Org. Proc. Res. Dev. 2005, 9, pages 51 to 56 (in particular pages 55 and 56) describes an alternative procedure, according to the following schema 1, wherein a protected 3-amino-4-picoline is converted to 3-amino-piperidine by means of total reduction of the pyridine ring.

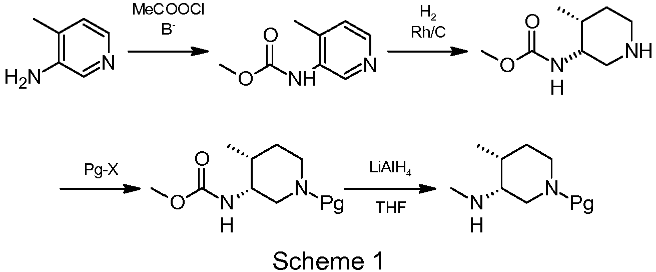

However, in this synthetic pathway, the rare and costly 3-amino-4-picoline is required as starting material. Besides, hydrogenation has to be carried out at high hydrogen pressure in order to achieve total reduction of the pyridine moiety to piperidine. The products of said synthesis are however racemic compounds.

WO 2007/012953, on example 3 at page 19, describes the last step of a further synthetic pathway in which 3-amino-4-picoline is used as starting material. As can be gathered from scheme 2, the pathway contains the steps of benzyl activation of pyridine ring and partial reduction using sodium borohydride. In the final step, asymmetric hydrogenation is carried out by means an optically active rhodium complex (formed by bis(1,5-cyclooctadiene)rhodium triflate and ferrocenyl phosphine Josiphos SL-J009-1™), in a mixture of tetrahydrofuran and ethanol to obtain (3*R*,4*R*)-(1-benzyl-4-methyl-piperidine-3-yl)-methylamine in 97% cis product in modest enantioselectivity of at best 66% e.e., and ratio Z/E, i.e. ratio between cis/trans diastereoisomers (abbreviated Dr), of 48.5.

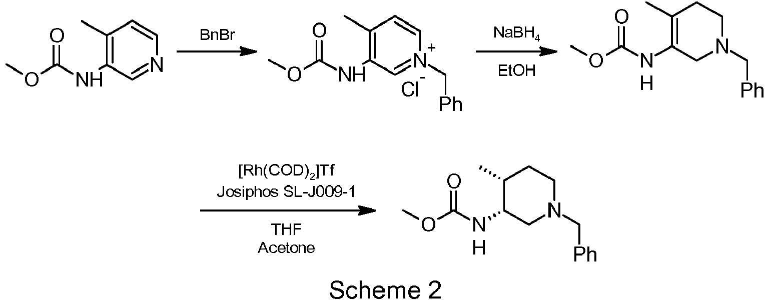

This potential prior art has not been confirmed by our experimentation. In particular, repeating the experiment 3 of WO 2007/012953, the (3*S*,4*S*)-(1-benzyl-4-methyl-piperidine-3-yl)-methylamine was isolated and not the alleged (3*R*,4*R*)-(1-benzyl-4-methyl-piperidine-3-yl)-methylamine as stated in said application, as confirmed by chiral HPLC analysis (see analytical method in the experimental part).

However, the claims from 44 to 47 of the application WO 2007/012953 appears to disclose a process for the preparation of enantiomerically enriched piperidine of formula: 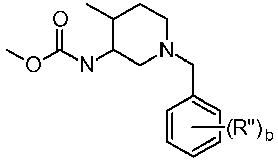 wherein R" in chosen from the group consisting of hydrogen, (C1-C6)alkyl and CF3 groups, b is an integer from 0 to 4, by asymmetrically hydrogenation of a tetrahydropyridine of formula:

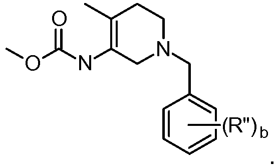

The object of the present invention is to provide an improved process for preparing 3-amino-piperidine compounds representing valuable key intermediates for the preparation of pharmaceutically active agents.

### Summary of invention

The problem addressed by the present invention is therefore that of providing an improved process for the preparation of a 3-(methylamino)-4-methylpiperidine moiety.

In particular, the problem of the present invention is to provide a better process for the preparation of a 3-(methylamino)-4-methylpiperidine moiety, with improvements especially in terms of enantiomeric excess (e.e.) and/or diastereomeric ratio and/or molar yield, or conversion.

This problem is solved by a process for the synthesis of 3-(methylamino)-4-methylpiperidine moiety as outlined in the annexed claims, whose definitions are integral part of the present description.

Further features and advantages of the process according to the invention will result from the description hereafter reported of examples of realization of the invention, provided as an indication of the invention.

### Detailed description of the invention

The present invention provides a process for the preparation of a compound of formula (II) or a salt thereof:

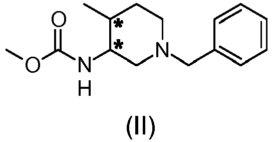

wherein the substituents at positions 3 and 4 of the piperidine ring are in a cis configuration, i.e. both the asymmetric carbons have *R* configuration or *S* configuration.

The process comprising the asymmetrical hydrogenation of the compound of formula (III) or a salt thereof:

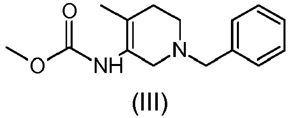

in a solvent, and the presence of a Rh(I) complex and of an optically active ferrocenyl phosphine.

In particular, the compound of formula (II) or a salt thereof has, at the asymmetric carbons marked with the symbol *, 3-*R* and 4-*R* optical configuration or 3-*S* and 4-*S* optical configuration or a mixture thereof, with the exclusion of the racemic mixture.

According to the invention, the solvent is 2,2,2-trifluoroethanol (TFE) or methanol, more preferably is TFE.

It has been indeed surprisingly found that said solvents allows the preparation of the compound (II) with enantiomeric excess (abbreviated e.e.) >67%, or at least 70%, and /or with very high conversions.

Furthermore, it has been indeed surprisingly found that the TFE solvent allows the preparation of the compound (II) with the completed conversion of the compound of formula (III) to the compound of formula (II), and with robust reproducibility of the results.

Moreover, it has been indeed surprisingly found that the TFE solvent allows the preparation of the compound (II) with a lower pressure and/or at lower temperature, and in much shorter reaction time.

The solvent can be used in amounts of at least 4 volumes, preferably at least 5 volumes, more preferably of between 8 and 12 volumes. Preferably, from 5 to 10 volumes of TFE or from 10 to 20 volumes of methanol are used.

The term "volume" referred to the solvent is to be understood as a volume per amount per weight amount of compound of formula (III).

The term "volume" thus means volume of solvent per unit of product, thus, for example, 1 volume is 1 Liter per 1 Kilo, or 1 mL for 1 gram, or 1 microliter per 1 milligram. Thus, 10 volumes means for example 10 liters per 1 Kilogram of substance.

The Rh(I) complex is a neutral complex of the general formula (IVa) or (IVb):

[RhLA]₂ (IVa)

[RhL₂A] (IVb)

wherein L represents a C₄-₁₂ diene or two C₂-₁₂ alkene molecules; A is chlorine, bromine, iodine, trifluoromethanesulfone, tetrafluoroborate or acetylacetonate. More preferably, L is norbornadiene or 1,5-cyclooctadiene and/or A is trifluoromethansulfone.

The optically active ferrocenyl phosphine is a compound of following formula (V): 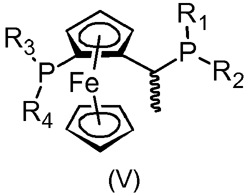 wherein R₁, R₂, R₃ and R₄ are independently selected between linear or branched C₁-₅ alkyl, unsubstituted aryl, substituted aryl with a linear or branched C₁-₅ alkyl group or is a cyclic C₅-₆ alkyl.

In the optically active ferrocenyl phosphine of formula (V) of the process of the invention , the linear or branched C₁₋₅ alkyl of R₁, R₂, R₃ and R₄, can be methyl, ethyl, n-propyl, isopropyl, n-butyl, isobutyl, sec-butyl, tert-butyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl.

The unsubstituted aryl group of R₁, R₂, R₃ and R₄ can be phenyl, furyl or naphthyl.

In the substituted aryl with a linear or branched C₁-₅ alkyl group of R₁, R₂, R₃ and R₄, the C₁₋₅ alkyl group is methylene, ethyl, n-propyl, isopropyl, n-butyl, isobutyl, sec-butyl, tert-butyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, methoxy, ethoxy, n-propoxy, isopropoxy, n-butoxy, sec-buthoxy, n-pentoxy, etc..

The cyclic C₅-₆ alkyl group of R₁, R₂, R₃ and R₄ can be cyclopentyl or cyclohexyl.

In a preferred embodiment, the optically active ferrocenyl phosphine compound of formula (V): 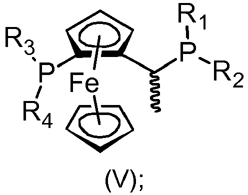 can be selected in a group comprising:
- (*R*)-1-[(*S_{P}*)-2-(Diphenylphosphino)ferrocenyl]ethyldi-tert-butyl phosphine with CAS [155830-69-6], is also sometimes named Josiphos SL-J002, having the following formula: 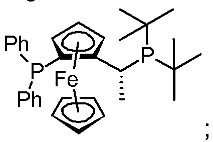
- (*S*)-1-[(*R_{P}*)-2-(Di-tert-butylphosphino)ferrocenyl]ethylbis(2-methyl phenyl)phosphine with CAS [849924-77-2], is also sometimes named Josiphos SL-J505-2, having the following formula: 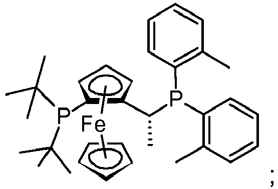
- (*S*)-1-{(*R_{P}*)-2-[Bis[4-(trifluoromethyl)phenyl]phosphino]ferrocenyl} ethyldi-tert-butylphosphine with CAS [849924-37-4], is also sometimes named Josiphos SL-J011-2, having the following formula: 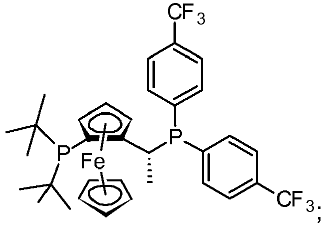
- (*S*)-1-[(*R_{P}*)-2-(Di-tert-butylphosphino)ferrocenyl]ethyldiphenyl phosphine with CAS [223121-01-5], is also sometimes named Josiphos SL-J502-2, having the following formula: 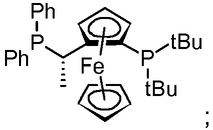
- (*R*)-1-[(*S_{P}*)-2-[Bis(4-methoxy-3,5-dimethylphenyl)phosphino]ferrocenyl} ethyldi-tert-butylphosphine with CAS [187733-50-2], is also sometimes named Josiphos SL-J013-1, having the following formula: 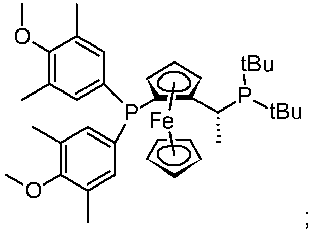
- (*S*)-1-[(*R_{P}*)-2-(diphenylphosphino)ferrocenyl]ethyldicyclohexyl phosphine with CAS [162291-02-3], is also sometimes named Josiphos SL-J001-2, having the following formula: 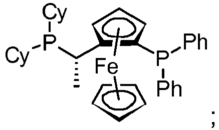
- (*S*)-1-[(*R_{P}*)-2-(dicyclohexyphosphino)ferrocenylethyl]diphenyl phosphine with CAS [162291-01-2], is also sometimes named Josiphos SL-J004-2, having the following formula: 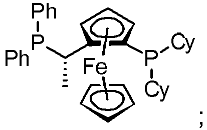
- (*R*)-1-[(*S_{P}*)-2-(Dicyclohexylphosphino)ferrocenyl]ethyldi-tert-butyl phosphine with CAS [158923-11-6], is also sometimes named Josiphos SL-J009-1, having the following formula: 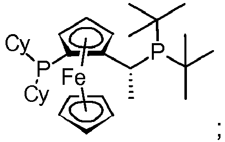
- (*R*)-1-{(*S_{P}*)-2-[Di(2-furyl)phosphino]ferrocenyl}ethyldi-tert-butyl phosphine with CAS [849924-41-0], is also sometimes named Josiphos SL-J212-1, having the following formula: 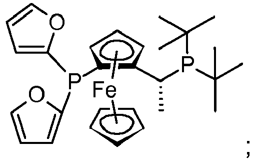
or their enantiomers.

More preferably, the ferrocenyl phosphine is (*R*)-1-[(*S*_{P})-2-(Di-*tert-*butylphosphino)ferrocenyl]ethylbis(2-methylphenyl) phosphine, having the formula (VI): 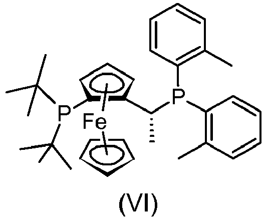 or its (*S,R_{P}*) enantiomer, since they provides the best results in terms of e.e..

It should be understood that, by using the (*S,Rp*)-enantiomer of the above ferrocenyl phosphine of formula (VI), the (3*S*,4*S*)-enantiomer of the compound of formula (II) is obtained, while the preferred (*R,Sp*)-enantiomer of the above ferrocenyl phosphine of formula (VI) gives the preferred opposite (3*R*,4*R*)-enantiomer of the compound of formula (II).

The rhodium (I) complex and the ferrocenyl phosphine are preferably used in a percent amount (w/w) of from about 0.5 to about 2%, more preferably about 1%, compared to the amount of compound of formula (III).

The term "about" in the whole context of the present invention means a variation of the indicated value of ±15%. For example, if "about 1%" of a compound is indicated, this means that an amount ranging from 0.85% to 1.15% can be used.

This reaction is preferably conducted at a temperature selected in the range of from 25°C to 70°C, preferably from 30°C to 60°C, and at a hydrogen pressure of from 2 and 20 bar, preferably from 3 to 15 bar. The reaction time is above 24 hours, preferably above 40 hours.

In particular, when TFE is used as the solvent, the reaction temperature is preferably selected in the range of from 30°C to 60°C, while when methanol is used as the solvent, the reaction temperature is preferably selected in the range of from 50°C to 70°C.

In particular, when TFE is used as the solvent, the reaction temperature is preferably selected in the range of from 30°C to 60°C, more preferably in the range of from 30°C to 40°C.

In a particularly preferred embodiment, when methanol is used as the solvent, the reaction temperature is preferably selected in the range of from 50°C to 70°C, more preferably in the range of from 55°C to 65°C, again more preferably at 60°C.

Moreover, preferably, when TFE is used as the solvent, the reaction pressure is selected in the range of from 2 to 15 bar, while when methanol is used as the solvent, the reaction pressure is selected in the range of from 10 to 20 bar.

In particular, when TFE is used as solvent, the reaction pressure is preferably selected in the range of from 2 to 15 bar, more preferably in the range from 2 to 5 bar, more preferably in the range of about 3 to about 4 bar, more preferably at about 3.5 bar.

In a particularly preferred embodiment, the inventive process comprises the hydrogenation of the compound of formula (III) to give the compound of formula (II), when TFE is used as solvent, is carried out at temperature in the range of from 30°C to 60°C and with a hydrogen pressure selected in the range from 2 to 5 bar.

Moreover, preferably, when TFE is used as the solvent, the inventive process comprises the hydrogenation of the compound of formula (III) to give the compound of formula (II), is carried out at temperature in the range of from 30°C to 40°C and with a hydrogen pressure selected in the range of about 3 to about 4 bar.

In a particularly preferred embodiment, the inventive process comprises the hydrogenation of the compound of formula (III) to give the compound of formula (II) in about 10 volumes of 2,2,2-trifluoroethanol, at temperature in the range of from 30°C to 40°C and with a hydrogen pressure selected in the range of about 3 to about 4 bar.

In a particularly preferred embodiment, the inventive process comprises the hydrogenation of the compound of formula (III) to give the compound of formula (II) in about 10 volumes of 2,2,2-trifluoroethanol, at temperature in the range of from 30°C to 40°C and with a hydrogen pressure selected in the range of about 3 to about 4 bar.

The reaction time to complete the reaction depending on the condition is 60 hours or less, e.g. 5 hours.

The process of the invention allows to obtain the compound of formula (II), either as a (3*S,4S*)- or (3*R*,4*R*) enantiomer, with an enantiomeric excess (e.e.) higher than 67%, preferably of at least 70%, more preferably of at least 75%.

The process of the invention may also comprise the following steps:
(i) reduction of the compound of formula (II), or salts thereof: 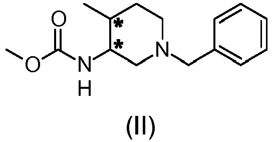
   wherein the asymmetric carbons marked with the symbol * have 3-*R* and 4-*R* optical configuration or 3-*S* and 4-*S* optical configuration or mixture thereof, with the exclusion of the racemic mixture;
   to give the compound 1-benzyl-N,4-dimethylpiperidin-3-amine of formula (VII) or salts thereof: 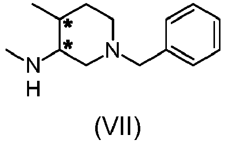 wherein the asymmetric carbons marked with the symbol * have 3-*R* and 4-*R* optical configuration or 3-*S* and 4-*S* optical configuration or mixture thereof, with the exclusion of the racemic mixture.
(ii) optionally, conversion of the compound of formula (VII), as the (*R,R*)-enantiomer, or salts thereof, of formula (VII-*RR*): 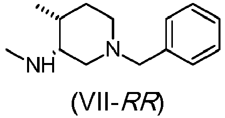 into Tofacitinib, or salts thereof, of formula (I):

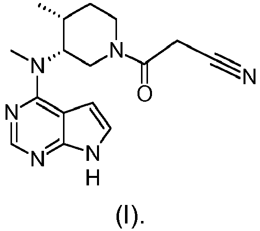

Step (i) can be performed by reacting the compound of formula (II) with an hydride in a solvent. Preferably, lithium aluminium hydride is used. Preferred reaction conditions provide for the use of an excess of hydride of at least 3 equivalents in a THF solvent at reflux temperature.

This reaction can be followed by the salification of the compound of formula (VII), for example with hydrogen chloride in a non-aqueous solvent.

The salification of the compound of formula (VII) with hydrogen chloride generates the compound of formula (VIII): 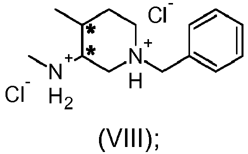 said bis-HCI salt of the compound (VII) wherein the asymmetric carbons marked with the symbol * have 3-*R* and 4-*R* optical configuration or 3-*S* and 4-*S* optical configuration or mixture thereof, allows an efficient purging of the undesired isomer, to obtain an efficient enrichment in terms of enantiomeric excess.

In particular, the compound (VIII) bis-HCI salt can be prepared in a solution of compound of formula (VII) in methanol by addition of hydrochloric acid. In particular, the addition of from 1% to 5% of water to the solution increases the enrichment in terms of enantiomeric excess.

Step (i) substantially retains the optical configuration of the starting compound of formula (II). This means that, if compound of formula (II) with an e.e. higher than 67% is reacted, a compound of formula (VII) with an e.e. higher than 67% will be obtained.

Step (ii) can be performed according to well known methods, for example those described in WO 2014/195978 in example from 1 to 6 (page from 25 to page 27). The conversion of the compound of formula (VII), as the (*R,R*)-enantiomer, or salts thereof, into Tofacitinib, or salts thereof, of formula (I) also substantially retains the enantiomeric excess of the starting compound of formula (VII).

Moreover, the compound of formula (III) can also be prepared as described in the WO 2007/012953 (see example 2).

All of the intermediates and compounds of the present invention in particular those of formula (I), (II), (III), (VII) can be in isolated or in not isolated form, from the reaction mixture wherein they are prepared.

According to the preferred embodiment, all of the intermediates and compounds isolated are typically in form of a solid.

The following scheme shows the overall process for preparing the compound of formula (VII) and (VIII) having 3*R* and 4*R* configuration.

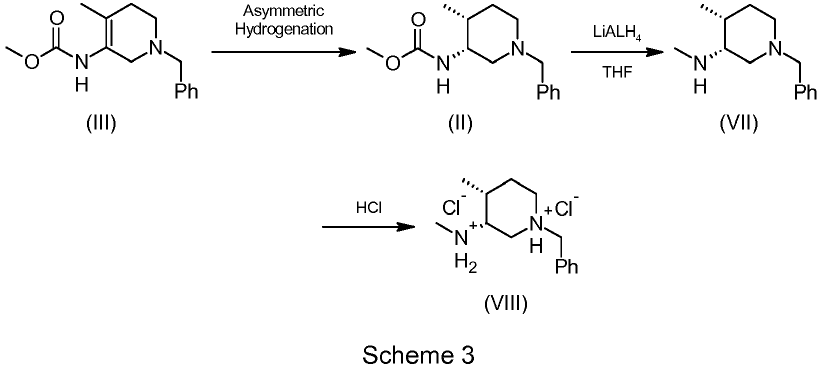

### EXPERIMENTAL PART

The Rh(I) complex and the ferrocenyl ligand, are reactants largely commercially available, for example, for supplied by: Sigma Aldrich (USA) or Alfa Aesar (Germany).

The starting material, i.e. the compound of formula (III) can be prepared according the teaching of international application publication No. WO 2007/012953 in the example 2 at pag. 19.

### Asymmetric hydrogenation.

### Example 1: Preparation of methyl (1-benzyl-4-methylpiperidin-3-yl)carbamate (II) - in methanol.

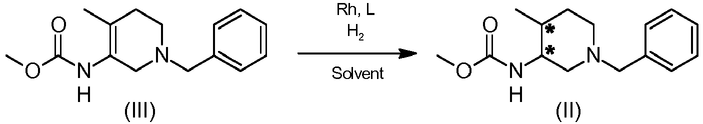

Six experiments have been carried out keeping constant the substrate concentration, temperature, catalyst and ligand loading and type, and solvent according to the conditions of the table below, as well as any other parameter/variable.

### Conditions

| **Rh(I) complex eq** | **Reaction T** | **Ligand Type** | **Ligand eq** |
|---|---|---|---|
| 0.01 eq | 60 °C | Josiphos SL-J505-2 | 0.01 eq |

### Procedure

To a 250 mL pressure vessel were added (1-benzyl-4-methyl-1,2,5,6-tetrahydropyidin-3-yl) carbamate (compound (III), 1.0 g, 3.84 mmol), bis(1,5-cyclooctadiene)rhodium(I) trifluoromethanesulfonate (18.0 mg, 0.0384 mmol, Aldrich 530840, CAS: 99326-34-8), (*S*)-1-[(*Rp*)-2-(di-tert-butylphosphino)ferrocenyl]ethylbis(2-methylphenyl)phosphine (22.0 mg, 0.0384 mmol, Aldrich 88756, CAS: 849924-77-2). The solids were purged with nitrogen (5x5 bar) then the methanol was added. The solution was purged with nitrogen (5x5 bar) followed by hydrogen. The reaction was heated and maintaining the hydrogen pressure. After the complete reaction, the mixture was cooled and purged with nitrogen (5x5 bar). An aliquot was removed for HPLC analysis to confirm full reaction conversion. The mixture was concentrated to dryness and methyl (1-benzyl-4-methylpiperidin-3-yl)carbamate (II) was isolated as an oil and analysed for chirality.

**Table 1: Comparison of the condition, with same ligand.**

| **Trial** | **Pressure (bar)** | **MeOH (V)** | **Time (h)** | **Conversion (%)** | **Yield (%)** | **Dr (cis/trans)** | **e.e.% (*S,S* isomer)** |
|---|---|---|---|---|---|---|---|
| 1 | 15 | 20 | 72 | 100% | / | 51.3 | 72% |
| 2 | 15 | 20 | 48 | 82% | 71% | 50.33 | 72% |
| 3 | 15 | 10 | 48 | 97% | 52% | >300 | 74.8% |
| 4 | 15 | 15 | 72 | 76% | / | >100 | 72.8% |
| 5 | 3.5 | 10 | 24 | 100% | 89% | 49.4 | 70.8% |
| 6 | 3.5 | 10 | 24 | 100% | 99% | 42 | 73.2% |

| | | | | | | | |
|---|---|---|---|---|---|---|---|
| Note: Dr is the diastereoisomeric ratio between the sum of the amount of the cis enantiomers over the sum of the amount of two trans isomer. | | | | | | | |

In others words Dr = ((3*S*,4*S*)+(3*R,*4*R*))/((3*R*,4*S*)+(3*S*,4*R*)).

### Example 2: Preparation of methyl (1-benzyl-4-methylpiperidin-3-yl)carbamate (II) - in TFE.

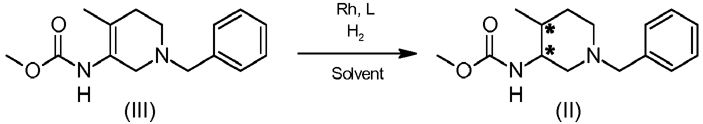

Ten experiments have been carried out keeping constant the substrate concentration, catalyst ligand loading and type, and solvent according to the conditions of the table below, as well as any other parameter/variable. For these examples TFE was chosen as the solvent.

### Conditions

| **Rh(I) complex eq** | **Ligand Type** | **Ligand eq** |
|---|---|---|
| 0.01 eq | Josiphos SL-J505-2 | 0.01 eq |

### Procedure

To a 250 mL pressure vessel were added (1-benzyl-4-methyl-1,2,5,6-tetrahydropyidin-3-yl) carbamate (compound (III), 1.0 g, 3.84 mmol), bis(1,5-cyclooctadiene)rhodium(I) trifluoromethanesulfonate (18.0 mg, 0.0384 mmol, Aldrich 530840, CAS: 99326-34-8), (*S*)-1-[(*Rp*)-2-(di-tert-butylphosphino)ferrocenyl]ethylbis(2-methylphenyl)phosphine (22.0 mg, 0.0384 mmol, Aldrich 88756, CAS: 849924-77-2). The solids were purged with nitrogen (5x5 bar) then the TFE was added. The solution was purged with nitrogen (5x5 bar) followed by hydrogen. The reaction was heated and maintaining the hydrogen pressure. After the complete reaction, the mixture was cooled and purged with nitrogen (5x5 bar). An aliquot was removed for HPLC analysis to confirm full reaction conversion. The mixture was concentrated to dryness and methyl (1-benzyl-4-methylpiperidin-3-yl)carbamate (II) was isolated as an oil and analysed for chirality.

**Table 2: Comparison of the condition, with same ligand.**

| **Trial** | **Pres. (bar)** | **Temp. (°C)** | **TFE (V)** | **Time (h)** | **Conv. (%)** | **Yield (%)** | **Dr (cis/trans)** | **e.e.% (*S,S* isomer)** |
|---|---|---|---|---|---|---|---|---|
| 1 | 15 | 60 | 10 | 72 | 100% | / | 32.9 | 74.3% |
| 2 | 3.5 | 30 | 10 | 72 | 100% | 87% | 182.9 | 82.9% |
| 3 | 3.5 | 40 | 10 | 72 | 100% | / | 97.1 | 76.4% |
| 4 | 3.5 | 30 | 10 | 48 | 98% | / | 152.2 | 80.7% |
| 5 | 3.5 | 60 | 10 | 6 | 100% | 95% | 50.6 | 74.1% |
| 6 | 3.5 | 60 | 5 | 6 | 100% | 94% | 61 | 74.8% |
| 7 | 3.5 | 50 | 5 | 6 | 100% | / | 101.7 | 78.9% |
| 8 | 2 | 60 | 5 | 6 | 100% | / | 33.9 | 73.6% |
| 9 | 2 | 50 | 10 | 24 | 98% | / | 88.5 | 79.4% |
| 10 | 2 | 40 | 10 | 24 | 98% | / | 41.6 | 75.7% |

### Example 3: Preparation of methyl ((3S,4S)-1-benzyl-4-methylpiperidin-3-yl)carbamate (II-SS)

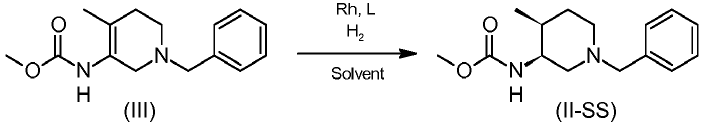

To a 250 mL pressure vessel were added (1-benzyl-4-methyl-1,2,5,6-tetrahydropyidin-3-yl)carbamate (III) (1.0 g, 3.84 mmol), bis(1,5-cyclooctadiene)rhodium(I) trifluoromethanesulfonate (18.0 mg, 0.0384 mmol, CAS: 99326-34-8), (*S*)-1-[(*Rp*)-2-(di-tert-butylphosphino)ferrocenyl]ethylbis(2-methylphenyl)phosphine (22.0 mg, 0.0384 mmol, CAS: 849924-77-2). The solids were purged with nitrogen (5x5 bar) then trifluoroethanol was added (10 mL, 10 V). The solution was purged with nitrogen (5x5 bar) followed by hydrogen (3.5 bar). The reaction was heated to 30°C maintaining the hydrogen pressure at 3.5 bar. After 72 h, the mixture was cooled and purged with nitrogen (5x5 bar). An aliquot was removed for HPLC analysis to confirm full reaction conversion. The mixture was concentrated to dryness and methyl-((3*S*,4*S*)-1-benzyl-4-methylpiperidin-3-yl)carbamate (II-*SS*) (990 mg, 87% yield, 82.9% e.e. and dr 182.9 chiral HPLC) was isolated as an oil.

Initial hydrogen pressure is 3.5 bar. As the reaction proceeds, pressure falls to 0 bar. The vessel is re-charged to 3.5 bar repeatedly until no further hydrogen consumption is observed (requiring approx. 5 re-charges).

### Example 4: Preparation of methyl ((3R,4R)-1-benzyl-4-methylpiperidin-3-yl)carbamate (II-RR)

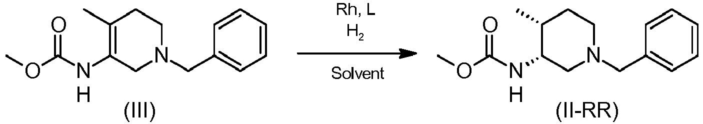

To a 15 mL pressure vessel were added (1-benzyl-4-methyl-1,2,5,6-tetrahydropyidin-3-yl)carbamate (III) (1.0 g, 3.84 mmol), bis(1,5-cyclooctadiene)rhodium(I) trifluoromethanesulfonate (18.0 mg, 0.0384 mmol, CAS: 99326-34-8) and (*R*)-1-[(*Sp*)-2-(di-tert-butylphosphino)ferrocenyl]ethyl-bis (2-methylphenyl)phosphine (22.0 mg, 0.0384 mmol, CAS: 849924-76-1) in trifluoroethanol (5 mL, 5 V). The solution was purged with nitrogen (5x5 bar) followed by hydrogen (3.5 bar). The reaction was heated at 30°C for 1 h then at 50°C for 5 h, maintaining the hydrogen pressure at 3.5 bar. The mixture was cooled and purged with nitrogen (5x5 bar). An aliquot was removed for HPLC analysis to confirm full reaction conversion (97% conv.). The mixture was concentrated to dryness to provide methyl ((3*R*,4*R*)-1-benzyl-4-methylpiperidin-3-yl)carbamate (II-*RR*) (1.08 g, quant. yield, 82.3% e.e. and dr 101.7 chiral HPLC) as an oil.

Initial hydrogen pressure is 3.5 bar. As the reaction proceeds, pressure falls to 0 bar. The vessel is re-charged to 3.5 bar repeatedly until no further hydrogen consumption is observed (requiring approx. 3 re-charges).

### Example 5: General preparation of 1-benzyl-N,4-dimethylpiperidin-3-amine (VII)

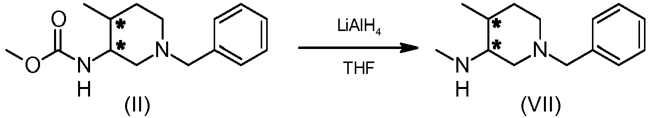

To a nitrogen-purged round-bottomed flask containing methyl (1-benzyl-4-methylpiperidin-3-yl)carbamate (II) (1 g, 4.28 mmol) in THF (10 mL) was added dropwise LiAlH₄ 1 M solution in THF (12.07 mL, 13.69 mmol) at 0 °C. The reaction mixture was stirred at 0°C for 5 min and was then heated at reflux for 2.5 h. After complete reaction, the mixture was cooled to 0-5 °C and water (2 mL) was slowly added over 5 min. The resulting suspension was filtered and the cake was washed with THF (4 mL). The combined filtrate and washings were concentrated to dryness to give 1-benzyl-N,4-dimethylpiperidin-3-amine (VII) as an oil.

### Example 6: Preparation of (3S,4S)-1-benzyl-N,4-dimethylpiperidin-3-amine (VII-SS)

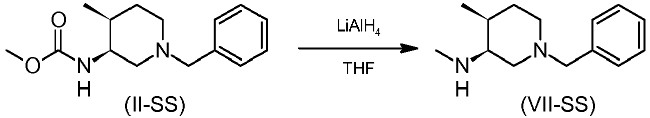

To a nitrogen-purged round-bottomed flask containing methyl ((3*S*,4*S*)-1-benzyl-4-methylpiperidin-3-yl)carbamate (II-*SS*) (0.99 g, 4.28 mmol) in THF (10 mL) was added dropwise LiAlH₄ 1 M solution in THF (12.07 mL, 13.69 mmol) at 0°C. The reaction mixture was stirred at 0°C for 5 min and was then heated at reflux for 2.5 h. After complete reaction, the mixture was cooled to 0-5°C and water (2 mL) was slowly added over 5 min. The resulting suspension was filtered and the cake was washed with THF (4 mL). The combined filtrate and washings were concentrated to dryness to give (3*S*,4*S*)-1-benzyl-N,4-dimethylpiperidin-3-amine (VII-*SS*) (0.512 g, crude, 59% yield) as an oil.

### Example 7: Preparation of (3R,4R)-1-benzyl-N,4-dimethylpiperidin-3-amine (VII-RR)

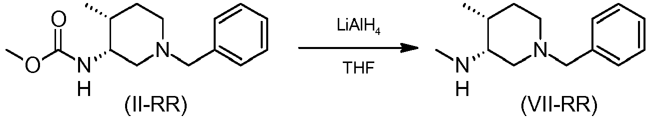

To a nitrogen-purged 100 mL round-bottomed flask containing methyl ((3*R*,4*R*)-1-benzyl-4-methylpiperidin-3-yl)carbamate (II-*RR*) (3.84 mmol) in THF (10 mL, 10 V) was added dropwise LiAlH₄ (1 M solution in THF, 12.29 mL, 12.29 mmol) at 0°C. The reaction mixture was stirred at 0°C for 20 min and was then heated at reflux for 2.5 h. After complete reaction, the mixture was cooled to 0-5°C and water (2 mL, 2 V) was slowly added over 5 min. The resulting suspension was filtered and the cake was washed with THF (4 mL). The combined filtrate and washings were concentrated to dryness to provide (3*R*,4*R*)-1-benzyl-N,4-dimethylpiperidin-3-amine (VII-*RR*) (0.625 g, 91.5% e.e. chiral GC) as an oil.

### Example 8: General preparation of 1-benzyl-N,4-dimethylpiperidin-3-amine dihydrochloride (VIII):

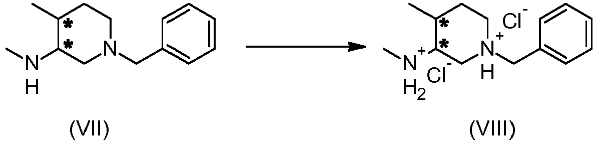

To a solution of 1-benzyl-N,4-dimethylpiperidin-3-amine (VII) (0.5 g, 2.34 mmol, in isopropanol (1.5 mL) was added a solution of 6 N HCI in isopropanol (1.25 mL, 3.2 eq). To the suspension formed was added heptane (1 mL) and the mixture was heated at reflux for 10 min. The suspension was stirred at RT for 30 min and the solid was filtered and washed with cold heptane (2x2 mL). The wet cake was dried at RT under vacuum to obtain 1-benzyl-N,4-dimethylpiperidin-3-amine dihydrochloride (VIII) as a pale solid.

### Example 9: Preparation of (3S,4S)-1-benzyl-N,4-dimethylpiperidin-3-amine dihydrochloride (VIII-SS)

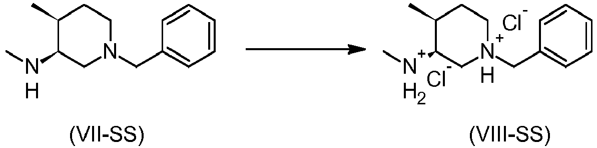

To a solution of (3*S*,4*S*)-1-benzyl-N,4-dimethylpiperidin-3-amine (VII-*SS*) (0.512 g, 2.34 mmol) in isopropanol (1.5 mL) was added a solution of 6 N HCI in isopropanol (1.25 mL, 3.2 eq). To the suspension formed was added heptane (1 mL) and the mixture was heated at reflux for 10 min. The suspension was stirred at RT for 30 min and the solid was filtered and washed with cold heptane (2x2 mL). The wet cake was dried at RT under vacuum to obtain (3*S*,4*S*)-1-benzyl-N,4-dimethylpiperidin-3-amine dihydrochloride (VIII-*SS*) (467 mg, 69% yield, 93.5% e.e. GC) as a pale solid.

### Example 10: Preparation of (3R,4R)-1-benzyl-N,4-dimethylpiperidin-3-amine dihydrochloride (VIII-RR)

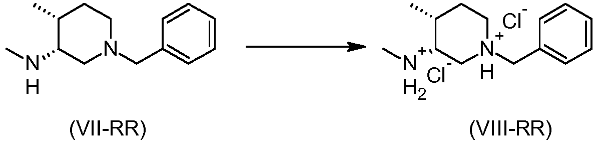

To a solution of (3*R*,4*R*)-1-benzyl-N,4-dimethylpiperidin-3-amine (VII-*RR*) (0.615 g, 2.82 mmol) in isopropanol (1.85 mL, 3 V) was added a solution of 6 N HCI in isopropanol (1.50 mL, 9.01 mmol, 3.2 eq). To the resulting suspension was added heptane (1 mL) and the mixture was heated at reflux temperature for 10 min. The suspension was stirred at RT for 30 min and the solid was filtered and washed with cold heptane (2x2 mL). The wet cake was dried at RT under vacuum to obtain (3*R*,4*R*)-1-benzyl-N,4-dimethylpiperidin-3-amine dihydrochloride (VIII-*RR*) (488 mg, 60% yield, 97.3% e.e. chiral GC) as a pale solid.

### Example 11: General recrystallization of 1-benzyl-N,4-dimethylpiperidin-3-amine dihydrochloride (VIII) in Methanol:

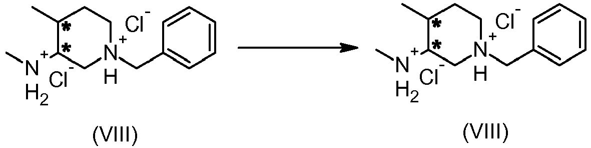

A suspension of 1-benzyl-N,4-dimethylpiperidin-3-amine dihydrochloride (VIII) (2.0 g) in MeOH (10 V) was heated at reflux until a solution was obtained. The mixture was cooled to RT and held for 12 h. The solid was filtered and washed with cold MeOH (2x1 V). The wet cake was dried at RT under vacuum to give the title salt (68% yield, 99% e.e.).

### Example 12: General recrystallization of 1-benzyl-N,4-dimethylpiperidin-3-amine dihydrochloride (VIII) in THF/H₂O (5%):

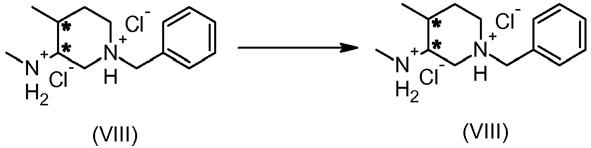

A suspension of 1-benzyl-N,4-dimethylpiperidin-3-amine dihydrochloride (VIII) (2.0 g) in THF/H2O(5%) (20 V) was heated at reflux until a solution was obtained. The mixture was cooled to RT and held for 12 h followed by 24 h at 0 °C. The solid observed in the aqueous layer was filtered and washed with cold THF (2x1 V). The wet cake was dried at RT under vacuum to give the title salt (24% yield, 98% e.e.).

### Example 13: Analytical methods

Analytical method for determining the e.e. of the present invention. The method monitoring the result of example from 1 to 4 and the chiral purity of the compound of formula (II), via HPLC, chromatographic conditions:
- Colum: Chiralcel OJ;
- Temp. Colum: 250°C;
- Mobile Phase: Heptane/IPA 95:5;
- Mode: Isocratic;
- Flow: 0.5 mL/min;
- UV Detector: 210 nm;
- Injection Volume: 5 µL;
- Analysis Time: 25 min;
- Diluent: Hepatane/lPa 1/1;

Retention time:
- Syn (*S,S*) = 6.8 min;
- Syn (*R,R*) = 9.2 min;
- Anti- (*R,S*) and (*S,R*) = 12.3 and 19.0 min (specific enantiomers not known).

Analytical method for determining the e.e. of the present invention. The method monitoring the result of example from 5 to 12 and the chiral purity of the compound of formula (VII) and (VIII), via GC, chromatographic conditions:
- Colum: Cyclosil-B;
- Temp. Inj: 230°C;
- Temp. Det.: 250°C;
- Oven Temperature program:
   ∘ start a 120°C;
   ∘ ramp at 3°C/min from 120°C to 205 °C;
   ∘ hold at 205°C for 2 minute;
   ∘ ramp at 10°C/min from 205°C to 220°C;
   ∘ hold at 220°C for 5 minutes.
- Flow: 1.0 mL/min;
- Split: 50:1
- UV Detector: 260 nm;
- Injection Volume: 1 µL;
- Analysis Time: 37 min;
- Diluent: dichloromethane;

Retention time:
- Syn (*S,S*) = 28 min;
- Syn (*R,R*) = 28.1 min.

## Claims

1. Process for the preparation of a compound of formula (II) or a salt thereof: 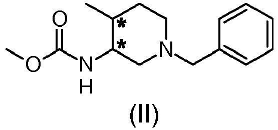
wherein the asymmetric carbons marked with the symbol * have 3-*R* and 4-*R* optical configuration or 3-*S* and 4-*S* optical configuration or mixture thereof, with the exclusion of the racemic mixture;
by asymmetrical hydrogenation of the compound of formula (III) or a salt thereof: 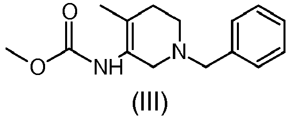 wherein said asymmetrical hydrogenation is carried out in a solvent and in presence of a Rh(I) complex and of an optically active ferrocenyl phosphine; wherein the solvent is 2,2,2-trifluoroethanol or methanol.

2. Process according to the claim 1, wherein the compound of formula (II) or salt thereof have an enantiomeric excess higher than 67%, or of at least 70%.

3. Process according to anyone of the claims from 1 to 2, wherein the Rh(I) complex is a neutral complex of the general formula (IVa) or (IVb):
[RhLA]₂ (IVa)
[RhL₂A] (IVb)
wherein L represents a C₄-₁₂ diene or two C₂-₁₂ alkene molecules; A is chlorine, bromine, iodine, trifluoromethanesulfone, tetrafluoroborate or acetylacetonate.

4. Process according to the claim 3, wherein L is norbornadiene or 1,5-cyclooctadiene.

5. Process according to anyone of the claims from 3 to 4, wherein A is trifluoromethansulfone.

6. Process according to any one of the claims from 1 to 5, wherein the ferrocenyl phosphine has the following formula (V): 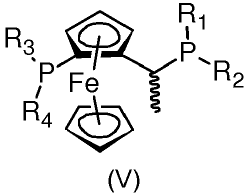 wherein R₁, R₂, R₃ and R₄ are independently selected among linear or branched C₁-₅ alkyl, unsubstituted aryl, substituted aryl with a linear or branched C₁-₅ alkyl group or is a cyclic C₅-₆ alkyl;

7. Process according to the claim 6, wherein ferrocenyl phosphine of formula (V) is (*R*)-1-[(*S*_{P})-2-(Di-*tert*-butylphosphino)ferrocenyl]ethylbis(2-methylphenyl) phosphine, having the formula (VI):

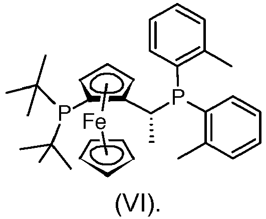

8. Process according to anyone of the claims from 1 to 7, wherein the asymmetrical hydrogenation is carried out at a temperature comprise between 30°C and 60°C wherein the solvent is 2,2,2-trifluoroethanol or, is carried out at a temperature comprised between 50°C and 70°C wherein the solvent is methanol.

9. Process according to anyone of the claims from 1 to 8, wherein the asymmetrical hydrogenation is carried in 2,2,2-trifluoroethanol and the pressure is comprised between 3 and 15 bar or, is carried in methanol and the pressure is comprised between 10 and 20 bar.

10. Process according to anyone of the claims from 1 to 9, wherein the asymmetrical hydrogenation is carried out in from 5 to 10 volumes of 2,2,2-trifluoroethanol or from 10 to 20 volumes of methanol.

11. Process according to anyone of the claims from 1 to 10, wherein the compound of formula (II) or a salt thereof, have the asymmetric carbons marked with the symbol * in the 3-*R* and 4-*R* optical configuration, and has the formula (II-*RR*): 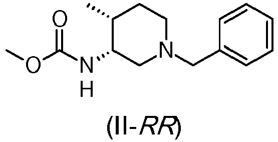 and has an enantiomeric excess higher than 67%, or at least 70%.

12. Process according to anyone of the claim from 1 to 11, comprising the further step of reduction of the compound of formula (II) or a salt thereof: 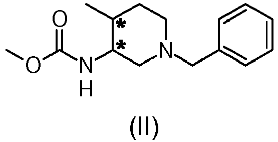
wherein the asymmetric carbons marked with the symbol * have 3-*R* and 4-*R* optical configuration or 3-*S* and 4-*S* optical configuration or mixture thereof, with the exclusion of the racemic mixture;
to give the compound 1-benzyl-N,4-dimethylpiperidin-3-amine of formula (VII) or a salt thereof: 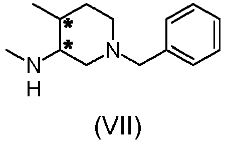 wherein the asymmetric carbons marked with the symbol * have 3-*R* and 4-*R* optical configuration or 3-*S* and 4-*S* optical configuration or mixture thereof, with the exclusion of the racemic mixture.

13. Process according to the claim 12, wherein the compounds of formula (II) and (VII) have the asymmetric carbons marked with the symbol * in the 3-*R* and 4-R optical configuration in an enantiomeric excess higher than 67%, or at least 70%, having the following formula:

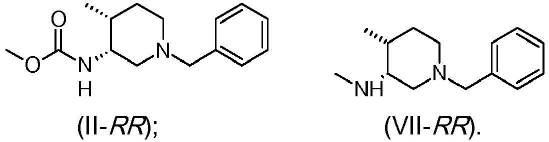

14. Process according to the claim 13, comprising the further steps of conversion of the compound of formula (VII-*RR*) or a salt thereof: 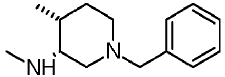 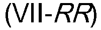 having an enantiomeric excess higher than 67%, or at least 70%, in the compound of formula (I): 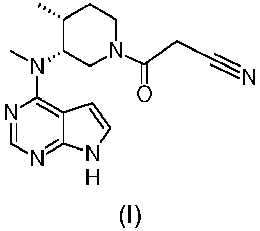 having an enantiomeric excess higher than 67%, or at least 70%.

15. Process for the preparation of Tofacitinib having the following formula or a salt thereof: 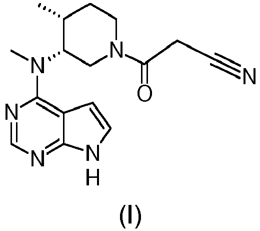 having an enantiomeric excess higher than 67%, or at least 70%, comprising the following steps:
A) preparation of the compound of formula (II-*RR*): 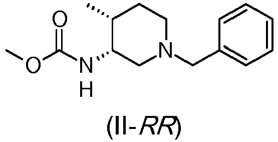 having an enantiomeric excess higher than 67%, or at least 70%,
according to the process of claim 11;
B) reduction of the compound of formula (II-*RR*) obtained in the step A) to give the compound (3*R*,4*R*)-1-benzyl-N,4-dimethylpiperidin-3-amine of formula (VII-*RR*) or a salt thereof: 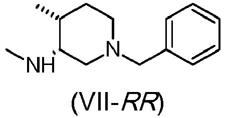 having an enantiomeric excess higher than 67%, or at least 70%;
C) conversion of the compound of formula (VII-*RR*) obtained in the step B) to Tofacitinib of formula (I) or a salt thereof, having an enantiomeric excess higher than 67%, or at least 70%.

16. Use of 2,2,2-trifluoroethanol for the preparation of the compound of formula (II) or a salt thereof: 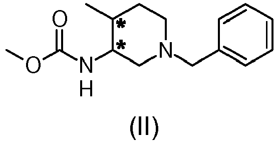
wherein the asymmetric carbons marked with the symbol * have 3-*R* and 4-*R* optical configuration or 3-*S* and 4-*S* optical configuration or mixture thereof, with the exclusion of the racemic mixture;
by asymmetrical hydrogenation of the compound of formula (III) or a salt thereof: 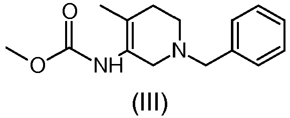 wherein said asymmetrical hydrogenation is carried out in a solvent and in presence of a Rh(I) complex and of an optically active ferrocenyl phosphine.

## Patentansprüche

1. Verfahren zur Herstellung einer Verbindung mit der Formel (II) oder eines Salzes davon: 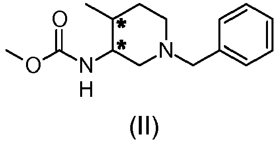
wobei die mit dem Symbol * markierten asymmetrischen Kohlenstoffatome die optische Konfiguration 3-*R* und 4-*R* oder die optische Konfiguration 3-*S* und 4-*S* oder eine Mischung davon aufweisen, unter Ausschluss der racemischen Mischung;
durch asymmetrische Hydrierung der Verbindung mit der Formel (III) oder eines Salzes davon: 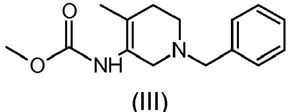 wobei die asymmetrische Hydrierung in einem Lösungsmittel und in Gegenwart eines Rh(I)-Komplexes und eines optisch aktiven Ferrocenylphosphins durchgeführt wird; wobei das Lösungsmittel 2,2,2-Trifluorethanol oder Methanol ist.

2. Verfahren nach Anspruch 1, wobei die Verbindung mit der Formel (II) oder das Salz davon einen Enantiomerenüberschuss höher als 67 % oder mindestens 70 % aufweist.

3. Verfahren nach einem der Ansprüche 1 bis 2, wobei der Rh(I)-Komplex ein neutraler Komplex mit der allgemeinen Formel (IVa) oder (IVb) ist:
[RhLA]₂ (IVa)
[RhL₂A] (IVb)
wobei L für ein C₄₋₁₂-Dien oder zwei C₂₋₁₂-Alkenmoleküle steht; A Chlor, Brom, Iod, Trifluormethansulfon, Tetrafluorborat oder Acetylacetonat ist.

4. Verfahren nach Anspruch 3, wobei L Norbornadien oder 1,5-Cyclooctadien ist.

5. Verfahren nach einem der Ansprüche 3 bis 4, wobei A Trifluormethansulfonat ist.

6. Verfahren nach einem der Ansprüche 1 bis 5, wobei das Ferrocenylphosphin die folgende Formel (V) aufweist: 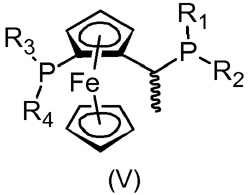 wobei R₁, R₂, R₃ und R₄ unabhängig ausgewählt sind aus linearem oder verzweigtem C₁₋₅-Alkyl, unsubstituiertem Aryl, substituiertem Aryl mit einer linearen oder verzweigten C₁₋₅-Alkylgruppe, oder ein cyclisches C₅₋₆-Alkyl ist;

7. Verfahren nach Anspruch 6, wobei das Ferrocenylphosphin mit der Formel (V) (*R*)-1-[(*S*_{P})-2-(Di-*tert*-butylphosphino)ferrocenyl]ethylbis(2-methylphenyl)phosphin mit der Formel (VI) ist:

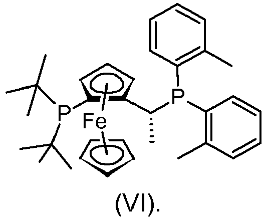

8. Verfahren nach einem der Ansprüche 1 bis 7, wobei die asymmetrische Hydrierung bei einer Temperatur durchgeführt wird, die zwischen 30 °C und 60 °C liegt, wobei das Lösungsmittel 2,2,2-Trifluorethanol ist, oder bei einer Temperatur durchgeführt wird, die zwischen 50 °C und 70 °C liegt, wobei das Lösungsmittel Methanol ist.

9. Verfahren nach einem der Ansprüche 1 bis 8, wobei die asymmetrische Hydrierung in 2,2,2-Trifluorethanol durchgeführt wird und der Druck zwischen 3 und 15 bar liegt, oder in Methanol durchgeführt wird und der Druck zwischen 10 und 20 bar liegt.

10. Verfahren nach einem der Ansprüche 1 bis 9, wobei die asymmetrische Hydrierung in 5 bis 10 Volumina 2,2,2-Trifluorethanol oder 10 bis 20 Volumina Methanol durchgeführt wird.

11. Verfahren nach einem der Ansprüche 1 bis 10, wobei in der Verbindung mit der Formel (II) oder einem Salz davon die mit dem Symbol * markierten asymmetrischen Kohlenstoffe in der optischen Konfiguration 3-*R* und 4-*R* vorliegen, und wobei sie die Formel (II-*RR*) aufweist: 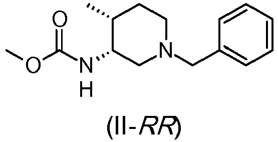 und einen Enantiomerenüberschuss höher als 67 % oder mindestens 70 % aufweist.

12. Verfahren nach einem der Ansprüche 1 bis 11, umfassend den weiteren Schritt des Reduzierens der Verbindung mit der Formel (II) oder eines Salzes davon: 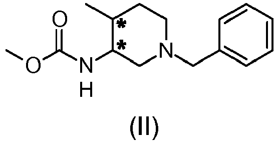
wobei die mit dem Symbol * markierten asymmetrischen Kohlenstoffatome die optische Konfiguration 3-*R* und 4-*R* oder die optische Konfiguration 3-*S* und 4-*S* oder eine Mischung davon aufweisen, unter Ausschluss der racemischen Mischung;
um die Verbindung 1-Benzyl-N,4-dimethylpiperidin-3-amin mit der Formel (VII) oder ein Salz davon zu ergeben: 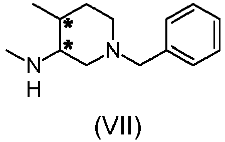 wobei die mit dem Symbol * markierten asymmetrischen Kohlenstoffatome die optische Konfiguration 3-*R* und 4-*R* oder die optische Konfiguration 3-*S* und 4-*S* oder eine Mischung davon aufweisen, unter Ausschluss der racemischen Mischung.

13. Verfahren nach Anspruch 12, wobei die Verbindungen mit der Formel (II) und (VII), in denen die mit dem Symbol * markierten asymmetrischen Kohlenstoffatome in der optischen Konfiguration 3-*R* und 4-*R* vorliegen, einen Enantiomerenüberschuss höher als 67 % oder mindestens 70 % aufweisen, mit der folgenden Formel:

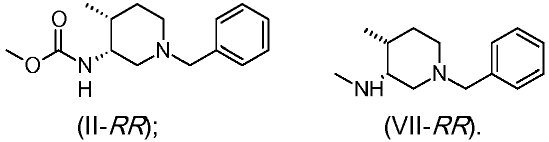

14. Verfahren nach Anspruch 13, umfassend den weiteren Schritt des Umwandelns der Verbindung mit der Formel (VII-*RR*) oder eines Salzes davon: 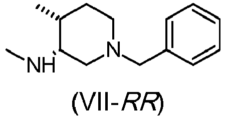 mit einem Enantiomerenüberschuss höher als 67 % oder mindestens 70 %, in die Verbindung mit der Formel (I): 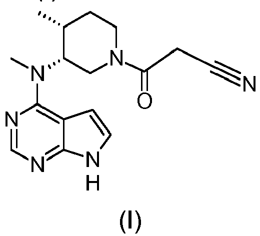 mit einem Enantiomerenüberschuss höher als 67 % oder mindestens 70 %.

15. Verfahren zur Herstellung von Tofacitinib mit der folgenden Formel oder einem Salz davon: 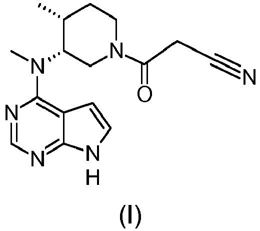 mit einem Enantiomerenüberschuss höher als 67 % oder mindestens 70 %, umfassend die folgenden Schritte:
A) Herstellen der Verbindung mit der Formel (II-*RR*): 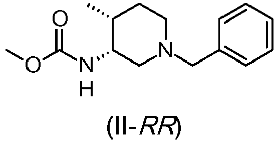 mit einem Enantiomerenüberschuss höher als 67 % oder mindestens 70 %, gemäß dem Verfahren von Anspruch 11;
B) Reduzieren der Verbindung mit der Formel (II-*RR*), die in Schritt A) erhalten wurde, um die Verbindung (3*R*,4*R*)-1-Benzyl-N,4-dimethylpiperidin-3-amin mit der Formel (VII-*RR*) oder ein Salz davon: 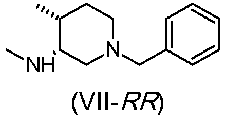 mit einem Enantiomerenüberschuss höher als 67 % oder mindestens 70 % zu ergeben;
C) Umwandeln der Verbindung mit der Formel (VII-*RR*), die in Schritt B) erhalten wurde, in Tofacitinib mit der Formel (I) oder ein Salz davon mit einem Enantiomerenüberschuss höher als 67 % oder mindestens 70 %.

16. Verwendung von 2,2,2-Trifluorethanol für die Herstellung der Verbindung mit der Formel (II) oder eines Salzes davon: 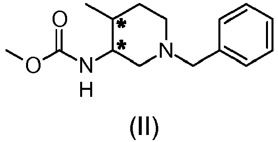
wobei die mit dem Symbol * markierten asymmetrischen Kohlenstoffatome die optische Konfiguration 3-*R* und 4-*R* oder die optische Konfiguration 3-*S* und 4-*S* oder eine Mischung davon aufweisen, unter Ausschluss der racemischen Mischung;
durch asymmetrische Hydrierung der Verbindung mit der Formel (III) oder eines Salzes davon: 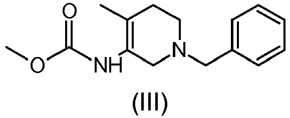 wobei die asymmetrische Hydrierung in einem Lösungsmittel und in Gegenwart eines Rh(I)-Komplexes und eines optisch aktiven Ferrocenylphosphins durchgeführt wird.

## Revendications

1. Procédé pour la préparation d'un composé de formule (II) ou d'un sel de celui-ci : 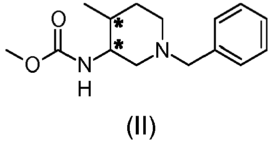
dans lequel les carbones asymétriques marqués avec le symbole * ont une configuration optique 3-*R* et 4-*R* ou une configuration optique 3-*S* et 4-*S* ou un mélange de celles-ci, à l'exclusion du mélange racémique ;
par hydrogénation asymétrique du composé de formule (III) ou d'un sel de celui-ci : 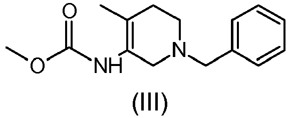 ladite hydrogénation asymétrique étant effectuée dans un solvant et en présence d'un complexe Rh(I) et d'une ferrocényle phosphine optiquement active ; le solvant étant du 2,2,2-trifluoroéthanol ou du méthanol.

2. Procédé selon la revendication 1, dans lequel le composé de formule (II) ou un sel de celui-ci a un excès énantiomérique supérieur à 67 %, ou d'au moins 70 %.

3. Procédé selon l'une quelconque des revendications 1 à 2, dans lequel le complexe Rh(I) est un complexe neutre de formule générale (IVa) ou (IVb) :
[RhLA]₂ (IVa)
[RhL₂A] (IVb)
dans laquelle L représente un diène en C₄-₁₂ ou deux molécules d'alcène en C₂-₁₂ ; A représente le chlore, le brome, l'iode, le trifluorométhanesulfone, le tétrafluoroborate ou l'acétylacétonate.

4. Procédé selon la revendication 3, dans lequel L est le norbornadiène ou le 1,5-cyclooctadiène.

5. Procédé selon l'une quelconque des revendications 3 à 4, dans lequel A est un trifluorométhanesulfone.

6. Procédé selon l'une quelconque des revendications 1 à 5, dans lequel la ferrocényle phosphine a la formule suivante (V) : 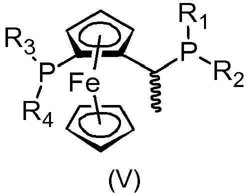 dans laquelle R₁, R₂, R₃ et R₄ sont indépendamment choisis parmi un alkyle en C₁-₅ linéaire ou ramifié, un aryle non substitué, un aryle substitué par un groupe alkyle en C₁-₅ linéaire ou ramifié ou est un groupe alkyle en C₅-₆ cyclique.;

7. Procédé selon la revendication 6, dans lequel la ferrocényle phosphine de formule (V) est (*R*)-1-[(*S*_{P})-2-(Di-tert-butylphosphino)ferrocényle]éthylbis(2-méthylphényl) phosphine, ayant la formule (VI) :

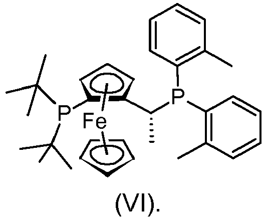

8. Procédé selon l'une quelconque des revendications 1 à 7, dans lequel l'hydrogénation asymétrique est effectuée à une température comprise entre 30° C et 60° C, le solvant étant le 2,2,2-trifluoroéthanol, ou est effectuée à une température comprise entre 50° C et 70° C, le solvant étant le méthanol.

9. Procédé selon l'une quelconque des revendications 1 à 8, dans lequel l'hydrogénation asymétrique est effectuée dans du 2,2,2-trifluoroéthanol et la pression est comprise entre 3 et 15 bars, ou est effectuée dans du méthanol et la pression est comprise entre 10 et 20 bars.

10. Procédé selon l'une quelconque des revendications 1 à 9, dans lequel l'hydrogénation asymétrique est effectuée dans 5 à 10 volumes de 2,2,2-trifluoroéthanol ou dans 10 à 20 volumes de méthanol.

11. Procédé selon l'une quelconque des revendications de 1 à 10, dans lequel le composé de formule (II) ou un sel de celui-ci, possède les carbones asymétriques marqués avec le symbole * dans la configuration optique 3-*R* et 4-*R*, et a la formule (II-*RR*) : 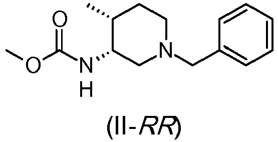 et a un excès énantiomérique supérieur à 67 %, ou au moins 70 %.

12. Procédé selon l'une quelconque des revendications 1 à 11, comprenant l'étape supplémentaire de réduction du composé de formule (II) ou d'un sel de celui-ci : 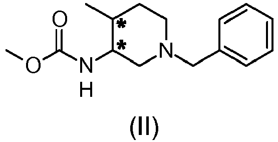
dans lequel les carbones asymétriques marqués avec le symbole * ont une configuration optique 3-*R* et 4-*R* ou une configuration optique 3-*S* et 4-*S* ou un mélange de celles-ci, à l'exclusion du mélange racémique ;
pour obtenir le composé 1-benzyl-N,4-diméthylpipéridin-3-amine de formule (VII) ou un sel de celui-ci : 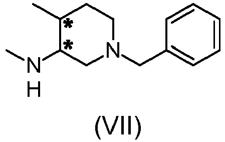 dans lequel les carbones asymétriques marqués avec le symbole * ont une configuration optique 3-*R* et 4-*R* ou une configuration optique 3-*S* et 4-*S* ou un mélange de celles-ci, à l'exclusion du mélange racémique.

13. Procédé selon la revendication 12, dans lequel les composés de formule (II) et (VII) ont les carbones asymétriques marqués avec le symbole * dans la configuration optique 3-*R* et 4-*R* dans un excès énantiomérique supérieur à 67 %, ou au moins 70 %, ayant la formule suivante :

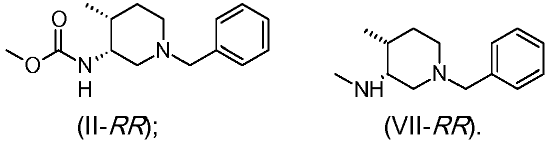

14. Procédé selon la revendication 13, comprenant les étapes supplémentaires de conversion du composé de formule (VII-*RR*) ou d'un sel de celui-ci : 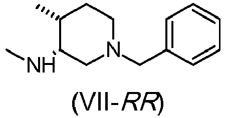 ayant un excès énantiomérique supérieur à 67 %, ou au moins 70 %, dans le composé de formule (I) : 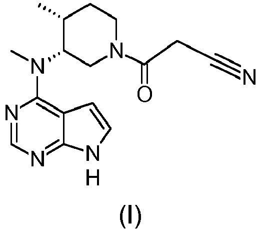 ayant un excès énantiomérique supérieur à 67 %, ou au moins 70 %.

15. Procédé pour la préparation de Tofacitinib ayant la formule suivante ou un sel de celui-ci : 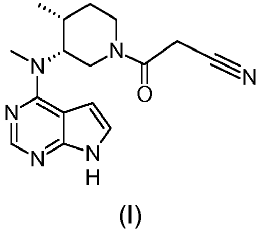 ayant un excès énantiomérique supérieur à 67 %, ou au moins 70 %, comprenant les étapes suivantes :
A) préparation du composé de formule (II-*RR*) : 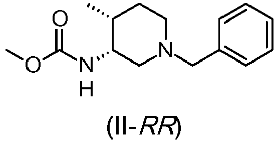 ayant un excès énantiomérique supérieur à 67 %, ou au moins 70 %, selon le procédé de la revendication 11 ;
B) réduction du composé de formule (II-*RR*) obtenu à l'étape A) pour obtenir le composé (3*R*,4*R*)-1-benzyl-N,4-diméthylpipéridin-3-amine de formule (VII-*RR*) ou un sel de celui-ci : 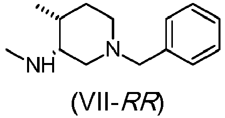 ayant un excès énantiomérique supérieur à 67 %, ou au moins 70 % ;
C) conversion du composé de formule (VII-*RR*) obtenu à l'étape B) en Tofacitinib de formule (I) ou un sel de celui-ci, ayant un excès énantiomérique supérieur à 67 %, ou au moins 70 %.

16. Utilisation de 2,2,2-trifluoroéthanol pour la préparation du composé de formule (II) ou d'un sel de celui-ci : 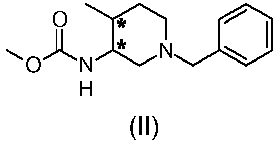
les carbones asymétriques marqués avec le symbole * ayant une configuration optique 3-*R* et 4-*R* ou une configuration optique 3-*S* et 4-*S* ou un mélange de celles-ci, à l'exclusion du mélange racémique ;
par hydrogénation asymétrique du composé de formule (III) ou d'un sel de celui-ci : 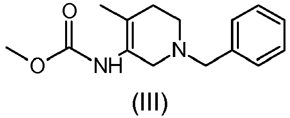 ladite hydrogénation asymétrique étant effectuée dans un solvant et en présence d'un complexe Rh(I) et d'une ferrocényle phosphine optiquement active.
